(19) Europäisches Patentamt
European Patent Office
Office européen des brevets

(11) EP 4 772 944 A1

(12) **EUROPEAN PATENT APPLICATION**
published in accordance with Art. 153(4) EPC

(43) Date of publication:
**08.07.2026 Bulletin 2026/28**

(21) Application number: **24859981.3**

(22) Date of filing: **30.08.2024**

(51) International Patent Classification (IPC):
***G03F 7/32*** (2006.01) ***C07C 211/63*** (2006.01)
***G03F 7/20*** (2006.01) ***G03F 7/26*** (2006.01)
***H01L 21/304*** (2006.01) ***H01L 21/306*** (2006.01)
***H01L 21/308*** (2006.01)

(52) Cooperative Patent Classification (CPC):
**C07C 211/63; G03F 7/20; G03F 7/26; G03F 7/32; H10P 50/00; H10P 50/691; H10P 52/00**

(86) International application number:
**PCT/JP2024/031290**

(87) International publication number:
**WO 2025/047956 (06.03.2025 Gazette 2025/10)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **01.09.2023 JP 2023142385**
**08.02.2024 JP 2024017580**
**24.04.2024 JP 2024070675**
**10.05.2024 JP 2024077289**

(71) Applicant: **TOKUYAMA CORPORATION**
**Yamaguchi 745-8648 (JP)**

(72) Inventors:
- **INOUE, Hiroshi**
**Shunan-shi, Yamaguchi 745-8648 (JP)**
- **KAWABATA, Yuichiro**
**Shunan-shi, Yamaguchi 745-8648 (JP)**
- **KOZAWA, Takahiro**
**Suita-shi, Osaka 565-0871 (JP)**
- **WANG, Jiahao**
**Suita-shi, Osaka 565-0871 (JP)**

(74) Representative: **Watermeyer, Nicholas**
**Withers & Rogers LLP**
**2 London Bridge**
**London SE1 9RA (GB)**

(54) **PHOTORESIST DEVELOPING SOLUTION**

(57) Provided is a photoresist developing solution comprising (A), (B), (C), and (D). (A) is a quaternary ammonium ion represented by formula (1), (B) is a halide ion, (C) is a hydroxide ion, and (D) is water. (In the formula, R1, R}2, R3, and R4 are each independently a C1-16 alkyl group; at least one of $R^1$, R2, R3, and R4 is a C2-16 alkyl group; and not all of $R^1$, R2, R3, and R4 are the same alkyl group.)

EP 4 772 944 A1

## Description

Technical Field

The present invention relates to a photoresist developer.

Background Art

Quaternary ammonium compounds are used in phase transfer catalysts, surfactants, disinfectants and the like. Among the quaternary ammonium compounds, tetramethylammonium hydroxide is particularly used as a kind of strongly basic organic alkali in aqueous alkaline solutions for semiconductors used in cleaning, etching, developers, and the like during semiconductor manufacturing.

For example, Patent Document 1 discloses a 2.35 wt.% aqueous tetramethylammonium hydroxide solution having a predetermined content of a metal ion and a halogen ion.

Prior Art Documents

Patent Document

Patent Document 1: JP H04-226466 A

Summary of Invention

Technical Problem

However, the present inventors have found that when an aqueous tetramethylammonium hydroxide (hereinafter, also described as TMAH) solution is used as a photoresist developer, the photoresist developer may have insufficient ability to develop a photoresist layer. When the ability to develop a photoresist layer is insufficient, the residue of the photoresist layer remains on a substrate during development, which may result in a defective pattern shape.

Accordingly, the present invention is directed to provide a photoresist developer which has a high ability to develop a photoresist layer and provides a good pattern shape. The present invention is also directed to provide a photoresist development method which provides a pattern having a good shape. In addition, the present invention is directed to provide a method for manufacturing a semiconductor device, which provides a good pattern shape.

Solution to Problem

The present inventors have made intensive studies to obtain a photoresist developer which provides a good pattern shape. As a result, the present inventors have found that using a photoresist developer containing a specific quaternary ammonium ion, a halide ion, a hydroxide ion, and water can provide a good pattern shape, and have completed the present invention.

That is, the configuration of the present invention is as follows.

Aspect 1. A photoresist developer containing the following (A), (B), (C) and (D):

(A) a quaternary ammonium ion represented by Formula (1) below;
(B) a halide ion;
(C) a hydroxide ion; and
(D) water

[Chem. 1]

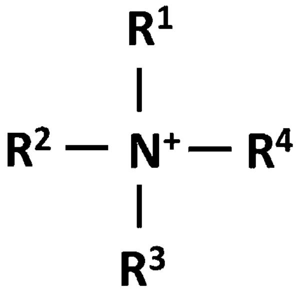

(1)

where $R^1$, $R^2$, $R^3$, and $R^4$ are each independently an alkyl group having carbon number from 1 to 16, provided that one or more alkyl groups of $R^1$, $R^2$, $R^3$, and $R^4$ have carbon number from 2 to 16, and $R^1$, $R^2$, $R^3$, and $R^4$ are not all the same alkyl group.

Aspect 2. The photoresist developer according to Aspect 1, wherein the photoresist developer has a surface tension of 73.0 mN/m or less at 25°C.

Aspect 3: The photoresist developer according to Aspect 1 or 2, wherein $R^1$, $R^2$, and $R^3$ in Formula (1) are methyl groups, and $R^4$ in Formula (1) is an alkyl group having carbon number from 2 to 16.

Aspect 4: The photoresist developer according to any one of Aspects 1 to 3, wherein the (B) halide ion is a chloride ion or a bromide ion.

Aspect 5. The photoresist developer according to any one of Aspects 1 to 4, wherein the halide ion is in an amount of 0.0000001 to 0.01 parts by weight when the hydroxide ion is in an amount of 1 part by weight, in the photoresist developer.

Aspect 6. The photoresist developer according to any one of Aspects 1 to 5, further containing one or more compounds selected from the group consisting of an alcohol and an amine.

Aspect 7. The photoresist developer according to any one of Aspects 1 to 6, the photoresist developer being a photoresist developer for developing an exposed photoresist layer containing a photosensitive resin.

Aspect 8. The photoresist developer according to any one of Aspects 1 to 7, wherein the photoresist developer contains one or more metal ions selected from the group consisting of a calcium ion, a sodium ion, a potassium ion, a chromium ion, a nickel ion, an iron ion, a lead ion, and an aluminum ion, and a content of each of the metal ions in the photoresist developer is independently 1 ppb or less.

Aspect 9. The photoresist developer according to any one of Aspects 1 to 8, the photoresist developer being a photoresist developer for developing a photoresist layer exposed using one or more excimer lasers selected from the group consisting of a KrF excimer laser and an ArF excimer laser.

Aspect 10. The photoresist developer according to any one of Aspects 1 to 9, wherein

in a dissolution rate evaluation test at 23°C of a photoresist layer containing a polyhydroxystyrene resin,
a ratio of a hydroxyl group and a tert-butoxycarbonyl group in the polyhydroxystyrene resin is 70 : 30 on a molar basis, the photoresist layer has a thickness of 200 nm,
a dissolution rate of the photoresist layer in a case of using the photoresist developer is 110% or more with respect to a dissolution rate of the photoresist layer in a case of using a photoresist developer containing tetramethylammonium hydroxide.

Aspect 11. The photoresist developer according to any one of Aspects 1 to 10, further containing a surfactant, wherein a concentration of the surfactant in the photoresist developer is less than 100 mass ppm.

Aspect 12. The photoresist developer according to any one of Aspects 1 to 11, further containing a tetramethylammonium ion.

Aspect 13. A photoresist development method, the method including a development step of developing an exposed photoresist layer using the photoresist developer according to any one of Aspects 1 to 12.

Aspect 14. A method for manufacturing a semiconductor device, the method including:

an exposure step of irradiating a photoresist layer on a substrate with light through a photomask having a predetermined pattern; and
a development step of developing the photoresist layer exposed in the exposure step and forming a resist pattern corresponding to the pattern of the photomask, wherein
in the development step, the exposed photoresist layer is developed using the photoresist developer according to any

one of Aspects 1 to 12.

## Effects of Invention

According to the present invention, a photoresist developer which provides a good pattern shape is provided. Further, a photoresist development method which provides a good pattern shape is provided. In addition, a method for manufacturing a semiconductor device, which provides a good pattern shape, is provided.

## Brief Description of Drawings

FIG. 1 is an explanatory view illustrating an electrolytic cell.

## Description of Embodiments

Embodiments of the present invention are described in detail below, but as long as the gist of the present invention is observed, the present invention is not limited to the details described below. In addition, the present invention can be modified and implemented in any manner that does not depart from the gist of the present invention.

In the present specification, a numerical range expressed using the term "to" refers to a range including the numerical values described before and after "to" as the lower limit and the upper limit, respectively. Thus, the expression "A to B" means A or more and B or less. Further, when numerical ranges are described in a stepwise manner, the upper limit and the lower limit of each numerical range can be optionally combined.

In addition, in the present specification, the description of a (meth)acrylic resin indicates a methacrylic resin and/or an acrylic resin. A monomer unit refers to a form obtained by a reaction of a monomer substance in a polymer.

A photoresist developer according to the present invention contains the following (A), (B), (C), and (D):

(A) a quaternary ammonium ion represented by Formula (1) below;
(B) a halide ion;
(C) a hydroxide ion; and
(D) water

[Chem. 2]

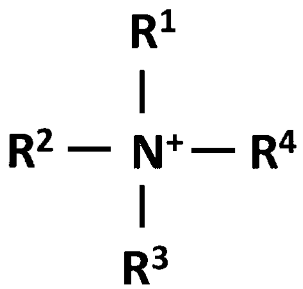

(1)

where $R^1$, $R^2$, $R^3$, and $R^4$ are each independently an alkyl group having carbon number from 1 to 16 (preferably from 1 to 8, and more preferably from 1 to 4), provided that one or more alkyl groups of $R^1$, $R^2$, $R^3$, and $R^4$ have carbon number from 2 to 16 (preferably from 2 to 8, and more preferably from 2 to 4), and $R^1$, $R^2$, $R^3$, and $R^4$ are not all the same alkyl group.

When the photoresist developer contains the quaternary ammonium ion represented by Formula (1), a better pattern shape is likely to be obtained than when an aqueous tetramethylammonium hydroxide solution is used as a photoresist developer. The reason why the good pattern shape is obtained is not clear, but the present inventors assume as follows.

In photolithography using a photoresist developer, a photoresist layer provided on a substrate is irradiated with light through a photomask having a predetermined pattern to be exposed. At this time, the photoresist included in the exposed region of the exposed photoresist layer undergoes a chemical reaction by the light. On the other hand, the photoresist contained in the unexposed region of the photoresist layer does not undergo a chemical reaction. The reactivity, solubility in a solvent, and the like change between the photoresist that has undergone a chemical reaction and the photoresist that has not undergone a chemical reaction, whereby the developability changes. Then, the exposed photoresist layer is developed using a photoresist developer, thereby forming a resist pattern corresponding to the

photomask.

In this way, the photoresist contained in the exposed region is chemically reacted by the light. However, this chemical reaction is less likely to proceed near the boundary of the photomask than near the center of the photomask. Thus, for example, when the photoresist is a positive photoresist, the photoresist near the boundary of the photomask does not have sufficient solubility in a developer, and a residue remains on the substrate, resulting in a defective pattern shape.

On the other hand, in the quaternary ammonium ion represented by the above Formula (1), $R^1$, $R^2$, $R^3$, and $R^4$ are each independently an alkyl group having carbon number from 1 to 16, $R^1$, $R^2$, $R^3$, and $R^4$ are not all the same alkyl group, and the carbon number of one or more alkyl groups of $R^1$, $R^2$, $R^3$, and $R^4$ is from 2 to 16. Thus, the quaternary ammonium ion has an asymmetric shape with respect to the nitrogen atom as the center, and at least one alkyl group has an alkyl group having carbon number from 2 to 16. Accordingly, in the quaternary ammonium ion represented by Formula (1), an alkyl group having a long carbon chain becomes lipophilic. As a result, for example, in a case where the photoresist is a positive photoresist, the affinity for a portion where a chemical reaction due to exposure is unlikely to proceed is increased. This improves the contact between the developer and the photoresist, and even the photoresist near the boundary of the photomask where the chemical reaction has not sufficiently proceeded can be sufficiently dissolved, including the influence of the affinity to the developer in terms of polarity.

As a result, the residue at the time of development is less likely to remain, which makes it possible to obtain a good pattern shape.

When the carbon numbers of $R^1$ to $R^4$ exceed the above range, the lipophilicity becomes excessively large, and the development rate may decrease. From the above viewpoint, the carbon number of one or more alkyl groups of $R^1$, $R^2$, $R^3$, and $R^4$ is also preferably from 2 to 16, more preferably from 3 to 16, still more preferably from 3 to 8, and most preferably from 3 to 4.

In Formula (1), $R^1$, $R^2$, $R^3$, and $R^4$ are not all the same alkyl group. In other words, compounds represented by the following (1) to (3) are preferred:

(1) a compound in which any three of $R^1$, $R^2$, $R^3$, and $R^4$ are the same alkyl group, and the remaining one is an alkyl group different therefrom;
(2) a compound in which any two of $R^1$, $R^2$, $R^3$, and $R^4$ are the same alkyl group, and the remaining two are alkyl groups different therefrom (the remaining two can be the same or different); and
(3) a compound in which all of $R^1$, $R^2$, $R^3$, and $R^4$ are different alkyl groups.

In the case of the above (1), examples of the compound can include compounds in which any three of $R^1$, $R^2$, $R^3$, and $R^4$ are the same alkyl group having carbon number from 1 to 16, the remaining one is an alkyl group having carbon number from 1 to 16, the first three and the remaining one are different alkyl groups, and either the first three or the remaining one has carbon number from 2 to 16. It is preferred that three of $R^1$, $R^2$, $R^3$, and $R^4$ in Formula (1) above be identical groups, and the remaining one be an alkyl group that has carbon number from 2 to 16 and is different from the three identical groups. In this case, the compound represented by Formula (1) has two types of alkyl groups. In this aspect, a compound in which any three of $R^1$, $R^2$, $R^3$, and $R^4$ are methyl groups and the remaining one alkyl group has carbon number from 2 to 16 is preferred. A compound in which any three of $R^1$, $R^2$, $R^3$, and $R^4$ are methyl groups and the remaining one alkyl group has carbon number from 2 to 16 is easy to serve as a surfactant described below. Among such compounds, a compound in which the remaining one alkyl group has carbon number from 2 to 8 is more preferred, and a compound in which the remaining one alkyl group has carbon number from 2 to 4 is still more preferred. In addition, the compound is also preferably a compound in which the remaining one alkyl group has carbon number from 3 to 16, more preferably a compound in which the remaining one alkyl group has carbon number from 3 to 8, and still more preferably a compound in which the remaining one alkyl group has carbon number from 3 to 4. When the carbon number is within the above range, the pattern shape is likely to be better.

In the case of the above (2), examples of the compound can include compounds in which any two of $R^1$, $R^2$, $R^3$, and $R^4$ are the same alkyl group having carbon number from 1 to 16, the remaining two are alkyl groups having carbon number from 1 to 16, which is different from the first two, the remaining two are the same or different alkyl groups, and either the first two or the remaining two have carbon number from 2 to 16. In this case, the compound represented by Formula (1) has two or three types of alkyl groups. In this aspect, a compound in which any two of $R^1$, $R^2$, $R^3$, and $R^4$ are methyl groups and the remaining two alkyl groups have carbon number from 2 to 16 is preferred. A compound in which any two of $R^1$, $R^2$, $R^3$, and $R^4$ are methyl groups is easy to serve as a surfactant described below. Among such compounds, a compound in which the remaining two alkyl groups have carbon number from 2 to 8 is more preferred, and a compound in which the remaining two alkyl groups have carbon number from 2 to 4 is still more preferred.

In the case of the above (3), examples of the compound can include compounds in which all of $R^1$, $R^2$, $R^3$, and $R^4$ are different alkyl groups, and at least one of them has carbon number from 2 to 16. In this case, the compound represented by Formula (1) has four types of alkyl groups. In this aspect, a compound in which any one of $R^1$, $R^2$, $R^3$, and $R^4$ is a methyl group and the remaining three alkyl groups have carbon number from 2 to 16 is preferred. A compound in which any one of

$R^1$, $R^2$, $R^3$, and $R^4$ is a methyl group is easy to serve as a surfactant described below. Among such compounds, a compound in which the remaining three alkyl groups have carbon number from 2 to 8 is more preferred, and a compound in which the remaining three alkyl groups have carbon number from 2 to 4 is still more preferred.

Specific examples of the quaternary ammonium ion represented by Formula (1) include an ethyltrimethylammonium ion, a diethyldimethylammonium ion, a triethylmethylammonium ion, a propyltrimethylammonium ion, and a butyltrimethylammonium ion. One of the quaternary ammonium ions can be used, or a plurality thereof can be used.

The halide ion is not particularly limited, and specific examples thereof include one or more selected from the group consisting of a chloride ion, a bromide ion, and an iodide ion. In addition, a chloride ion or a bromide ion is preferred.

In the photoresist developer, the amount in parts by weight of the halide ion when the weight of the hydroxide ion is in an amount of 1 part by weight is not particularly limited, but, preferably, is in an amount of from 0.0000001 to 0.01 parts by weight, more preferably from 0.0000001 to 0.001 parts by weight, and still more preferably from 0.0000001 to 0.0005 parts by weight. The part by weight of the halide ion varies greatly depending on the method for manufacturing the photoresist developer, and a solution having a reduced amount of halide ion can be prepared by using a manufacturing method by electrolysis in particular. The adjustment can be made by selecting the structure of anelectrolytic cell and a cation exchange membrane used for electrolysis and changing conditions such as a current density.

When the photoresist developer contains a large amount of halide ions, the halide ions may remain on the surface of the semiconductor when a photoresist layer is developed. In such a case, a scum may be generated at the time of development, which may deteriorate the physical properties of the semiconductor.

When the part by weight of the halide ion is within the above range, the pattern shape at the time of development is easily improved in a case of using the photoresist developer.

The part by weight of the halide ion in the photoresist developer can be analyzed by ion chromatography, and analysis by a neutralization method is effective particularly in a case where the photoresist developer has a high concentration. Specifically, before the photoresist developer is introduced into the anion ion chromatography, cations in the photoresist developer are exchanged with hydrogen ions to convert hydroxide ions into water. Thus, the hydroxide ions can be removed. Further, the sensitivity is enhanced by concentration. By using this method, it is possible to measure the concentration of a low-concentration halide ion.

The photoresist developer includes a hydroxide ion. The fact that the photoresist developer contains the quaternary ammonium ion represented by Formula (1) and the hydroxide ion can also be said to indicate that the photoresist developer contains a quaternary ammonium hydroxide (hydroxide of the quaternary ammonium ion represented by Formula (1)) containing the quaternary ammonium ion represented by Formula (1) and the hydroxide ion.

The photoresist developer contains the quaternary ammonium ion represented by Formula (1), and thus can be used as a photoresist developer.

Concentrations of the quaternary ammonium ion represented by Formula (1) and the hydroxide ion in the photoresist developer are not particularly limited, and a photoresist developer containing a quaternary ammonium hydroxide at a concentration of from 1.0 to 65.0 mass% is preferably manufactured. For example, the quaternary ammonium hydroxide is more preferably used at a concentration of from 1.0 to 10.0 mass%, and is still more preferably used at a concentration of from 1.0 to 7.0 mass%. In addition, the concentration is more preferably from 1.0 to 4.5 mass%.

The quaternary ammonium hydroxide is preferably used at a molarity of from 0.10 to 1.0 mol/L, is more preferably used at a molarity of from 0.10 to 0.50 mol/L, and is still more preferably used at a molarity of from 0.10 to 0.33 mol/L.

Further, a high-concentration photoresist developer containing a high-concentration hydroxide of the quaternary ammonium ion represented by Formula (1) can be diluted and used. With the high concentration, the volume during transportation can be reduced, which can suppress the transportation cost. Even when the photoresist developer is contaminated when it is filled in a container or transported, the influence of contamination can be reduced by the extent of dilution, as compared with a case where the dilution is not performed.

The concentration of the hydroxide of the quaternary ammonium ion represented by Formula (1) in the high-concentration photoresist developer before dilution is preferably from 10.0 to 50.0 mass%, and more preferably from 20.0 to 40.0 mass%. The concentration of the quaternary ammonium hydroxide can be confirmed by neutralization titration with an acid or the like.

The concentration of the quaternary ammonium ion represented by Formula (1) in the photoresist developer is preferably from 0.8 to 52.9 mass%. In addition, the concentration is more preferably from 0.8 to 8.2 mass%, and still more preferably from 0.8 to 5.7 mass%.

The concentration of the hydroxide of the quaternary ammonium represented by Formula (1) in the high-concentration photoresist developer is preferably from 8.0 to 40.7 mass%, and more preferably from 16.2 to 32.5 mass%.

The concentration of the hydroxide ion in the photoresist developer is preferably from 0.15 to 12.2 mass%. In addition, the concentration is more preferably from 0.15 to 1.9 mass%, and still more preferably from 0.15 to 1.3 mass%.

The concentration of the hydroxide ion in the high-concentration photoresist developer is preferably from 1.9 to 9.4 mass%, and more preferably from 3.7 to 7.5 mass%.

The photoresist developer contains water. A form of water is not particularly limited. Known water can optionally

be used, and it is particularly preferable that the water be ultrapure water in which metal impurities are decreased. The content of water in the photoresist developer is not particularly limited, but is preferably from 90.0 to 99.0 mass%, more preferably from 93.0 to 99.0 mass%, and still more preferably from 95.5 to 99.0 mass%.

The photoresist developer can contain one or more compounds selected from the group consisting of an alcohol and an amine. A concentration of the one or more compounds selected from the group consisting of an alcohol and an amine in the photoresist developer is not particularly limited, but is preferably from 0.1 to 10000 mass ppm, and more preferably from 0.1 to 100 mass ppm.

The alcohol is not particularly limited, and a known alcohol can be used. Examples of the alcohol include primary alcohols such as methanol, ethanol, and 1-propanol, secondary alcohols such as 2-propanol and 2-butanol, and tertiary alcohols such as 2-methyl-2-propanol. The number of hydroxyl groups in the alcohol is not particularly limited, and a monohydric alcohol can be used, or a dihydric or higher alcohol can be used. Among them, an alcohol having carbon number of 16 or less (more preferably carbon number from 2 to 8, and still more preferably carbon number from 2 to 4) is preferred from the viewpoint of increasing the possibility of causing adsorption inhibition or the like during development when the alcohol has a long chain.

The amine is not particularly limited, and a known amine can be used. Examples of the amine include primary amines such as methylamine, secondary amines such as dimethylamine, and tertiary amines such as trimethylamine. The number of amino groups in the amine is not particularly limited, and a monoamine can be used, or a diamine or higher amine can be used. Among them, an amine having carbon number of 16 or less (more preferably carbon number from 2 to 8, and still more preferably carbon number from 2 to 4) is preferred from the viewpoint that the amine having a long chain increases the possibility of causing adsorption inhibition or the like during development.

It is desirable that the photoresist developer contains one or more metal ions selected from the group consisting of a calcium ion, a sodium ion, a potassium ion, a chromium ion, a nickel ion, an iron ion, a lead ion, and an aluminum ion, and the content of each metal ion in the photoresist developer is independently a specific concentration or less. Among them, it is more desirable that the concentration of each of a chromium ion, a nickel ion, an iron ion, and an aluminum ion is a specific concentration or less. When the metal ions are reduced, metal contamination on the surface of the substrate after development is reduced, which makes the malfunction rate of the semiconductor likely to be reduced.

Further, the content of each metal ion in the photoresist developer is independently preferably 1 ppb or less, more preferably 0.5 ppb or less, and still more preferably 0.2 ppb or less. The lower limit is not particularly limited, and can be 0.1 ppt or more and 1 ppb or less, 0.1 ppt or more and 0.5 ppb or less, or 0.1 ppt or more and 0.2 ppb or less.

The metal ion concentration can be measured by inductively coupled plasma-mass spectrometry (ICP-MS).

A metal ion may be mixed in by contamination from a manufacturing raw material, a liquid contact portion, or the like. Thus, in a case where the content of each metal ion is large, the content can be reduced by purification with an ion exchange resin or the like.

The photoresist developer preferably has low toxicity. For example, in the case where the photoresist developer contains the quaternary ammonium ion represented by Formula (1), the photoresist developer is less toxic than in a case where an aqueous tetramethylammonium hydroxide solution is used as the photoresist developer.

As evaluation of the toxicity of the photoresist developer, a median lethal dose (LD50) value converted assuming that an amount of hydroxide of a quaternary ammonium ion is 100 mass%, in a toxicity test using a rat based on an acute oral toxicity test in accordance with OECD Test Guideline 420, is preferably more than 50 mg/kg. As a result, the safety in use is higher and the environmental load is further reduced. In addition, in the GHS classification, in a case where the LD50 value is 50 mg/kg or less, it falls under Category 2, and regulations in storage, transportation, and the like are strict, and in a case where the LD50 value is 5 mg/kg or less, it falls under Category 1, and the regulations are further strict. Thus, the LD50 value is preferably more than 50 mg/kg. The LD50 value is more preferably more than 50 mg/kg and 10000 mg/kg or less, still more preferably more than 300 mg/kg and 10000 mg/kg or less, and particularly preferably more than 2000 mg/kg and 10000 mg/kg or less.

The LD50 value can be adjusted by changing the type of the quaternary ammonium ion represented by Formula (1).

From the viewpoint of the toxicity, for example, it is preferable to use a quaternary ammonium ion in which $R^1$, $R^2$, and $R^3$ in Formula (1) each are an alkyl group having carbon number of 1, and $R^4$ is an alkyl group having carbon number from 2 to 4 (more preferably 3 to 4). More specifically, an ethyltrimethylammonium ion, a propyltrimethylammonium ion, and a butyltrimethylammonium ion are preferred, and a propyltrimethylammonium ion and a butyltrimethylammonium ion are more preferred.

The photoresist developer can also include a surfactant. The concentration of the surfactant in the photoresist developer is preferably less than 100 mass ppm. The photoresist developer does not need to include a surfactant.

The quaternary ammonium ion represented by Formula (1) contained in the photoresist developer is asymmetric with respect to the nitrogen atom as the center, and one or more alkyl groups of $R^1$, $R^2$, $R^3$, and $R^4$ have carbon number from 2 to 16, and thus, it is considered that the quaternary ammonium ion itself also serves as a surfactant. Specifically, a portion of the alkyl group having large carbon number becomes lipophilic, and a portion having small carbon number

becomes hydrophilic. Accordingly, the quaternary ammonium ion represented by Formula (1) has a lipophilic moiety and a hydrophilic moiety in the ion structure, and is considered to serve as a surfactant.

Thus, the photoresist developer does not need to contain a surfactant at all, and even when it contains a surfactant, the concentration thereof can be as low as less than 100 mass ppm. The concentration of the surfactant is more preferably less than 50 mass ppm, still more preferably less than 30 mass ppm, and particularly preferably less than 10 mass ppm. When the photoresist developer does not contain a surfactant, or when the concentration thereof is less than 100 mass ppm even if it contains a surfactant, for example, foaming of the photoresist developer can be suppressed, and impurities derived from the surfactant can be suppressed. In addition, in a case where the quaternary ammonium hydroxide is recovered from the used developer by separation, purification, or the like and is repeatedly used, when a surfactant is required as the developer, it is necessary to add the surfactant again. In this case as well, the amount of the surfactant to be added again can be suppressed when the surfactant concentration is low. In the case where the photoresist developer does not contain a surfactant, the recovered liquid can be used as it is as the developer.

The lower limit of the surfactant is not particularly defined, but from the viewpoint of exhibiting the above effect, the surfactant is preferably contained in an amount of 0.01 mass ppm or more, or 0.1 mass ppm or more. That is, the concentration of the surfactant can be, for example, 0.01 mass ppm or more and less than 100 mass ppm, 0.01 mass ppm or more and less than 50 mass ppm, 0.01 mass ppm or more and less than 30 mass ppm, 0.01 mass ppm or more and less than 10 mass ppm, or 0.1 mass ppm or more and less than 10 mass ppm.

The surfactant is a compound having a hydrophilic group and a hydrophobic group (lipophilic group) in one molecule, and examples thereof include a nonionic surfactant, an anionic surfactant, a cationic surfactant, and an amphoteric surfactant.

Note that the surfactant is a compound different from the above-described compound that may be contained in the photoresist developer.

The photoresist developer can contain a tetramethylammonium ion. The present inventors have found that in a case where the photoresist developer contains only a tetramethylammonium ion as the quaternary ammonium ion, a large amount of a surfactant may be required. The reason for this is presumed as follows.

For example, in a case where the photoresist developer contains a quaternary ammonium ion and the quaternary ammonium ion is a tetramethylammonium ion, the quaternary ammonium ion has only a methyl group having small carbon number. As a result, the tetramethylammonium ion becomes hydrophilic as a whole. That is, tetramethylammonium does not exhibit the effect of a surfactant.

However, the quaternary ammonium ion represented by Formula (1) contained in the photoresist developer according to the present invention is considered to also serve as a surfactant by itself as described above. Accordingly, even in a case where tetramethylammonium, which does not exhibit the effect of the surfactant, is contained, the amount of the surfactant can be reduced.

In addition, when the photoresist developer contains the quaternary ammonium ion represented by Formula (1) and further contains a tetramethylammonium ion, the development performance of the photoresist developer is likely to be excellent. As described above, the quaternary ammonium ion represented by Formula (1) is considered to also serve as a surfactant. Thus, even in a case where tetramethylammonium is contained, the development performance is likely to be improved. At this time, as described above, the photoresist developer does not need to contain a surfactant.

In a case where the photoresist developer includes a tetramethylammonium ion, the photoresist developer contains a tetramethylammonium ion and a hydroxide ion. The fact that the photoresist developer contains a tetramethylammonium ion and a hydroxide ion can also be said to indicate that the photoresist developer contains tetramethylammonium hydroxide containing a tetramethylammonium ion and a hydroxide ion.

The concentration of a tetramethylammonium ion in the photoresist developer is not particularly limited, and a photoresist developer containing tetramethylammonium hydroxide at a concentration of from 1.0 to 30.0 mass% is preferably manufactured. For example, the tetramethylammonium hydroxide is more preferably used at a concentration of from 0.1 to 5.0 mass%, and is still more preferably used at a concentration of from 0.5 to 3.0 mass%.

The tetramethylammonium hydroxide is preferably used at a molarity of from 0.01 to 0.55 mol/L, is more preferably used at a molarity of from 0.05 to 0.33 mol/L, and is still more preferably used at a molarity of from 0.1 to 0.27 mol/L.

The concentration of the tetramethylammonium ion in the photoresist developer is preferably from 0.08 to 4.0 mass%. In addition, the concentration is more preferably from 0.4 to 2.4 mass%, and still more preferably from 0.8 to 2.0 mass%.

The total concentration of the tetramethylammonium ion and the quaternary ammonium ion represented by Formula (1) in the photoresist developer is preferably from 0.5 to 6.0 mass%. In addition, the total concentration is more preferably from 1.0 to 5.0 mass%, and still more preferably from 1.2 to 4.0 mass%.

The total concentration of tetramethylammonium hydroxide and the hydroxide of the quaternary ammonium ion represented by Formula (1) in the photoresist developer is preferably from 0.4 to 5.0 mass%. In addition, the total concentration is more preferably from 0.8 to 4.0 mass%, and still more preferably from 1.0 to 3.2 mass%.

The total concentration of the tetramethylammonium ion and the quaternary ammonium ion represented by Formula (1) in the high-concentration photoresist developer is preferably from 10.0 to 50.0 mass%. In addition, the total concentration is more preferably from 15.0 to 40.0 mass%, and still more preferably from 20.0 to 30.0 mass%.

As described above, the quaternary ammonium ion represented by Formula (1) is considered to also serve as a surfactant. As a result, the photoresist developer has a lower surface tension than that of an aqueous tetramethylammonium hydroxide solution. As a result, the wettability to the photoresist during development is likely to be improved. Also in a case where the photoresist developer contains a tetramethylammonium ion, the surface tension is lowered as compared with the aqueous tetramethylammonium hydroxide solution, which improves the development characteristics.

The surface tension is measured with a surface tensiometer DY-300 (available from Kyowa Interface Science Co., Ltd). The surface tension of the photoresist developer at 25°C is preferably 73.0 mN/m or less, more preferably 72.0 mN/m or less, and still more preferably 71.0 mN/m or less. The measurement is performed in accordance with "Test Method by Wilhelmy Surface Tensiometer" of JIS2241.

The photoresist developer of the present invention has a high ability to develop a photoresist layer and provides a good pattern shape.

Note that the photoresist developer according to the present invention can contain a known additive used in developers in the related art as appropriate within a range not impairing the effects of the present invention. Examples of such an additive include a wetting agent, a stabilizer, and dissolution aid. Each of these can be added alone, or two or more thereof can be added in combination.

A method for manufacturing a photoresist developer is not particularly limited, but from the viewpoint of suppressing the halide ion concentration, it is preferable to obtain a photoresist developer by electrolyzing an aqueous quaternary ammonium halide solution. That is, the method preferably includes an electrolysis step of electrolyzing the aqueous quaternary ammonium halide solution. Specifically, the method preferably includes an electrolysis step of electrolyzing an aqueous quaternary ammonium halide solution containing the following (A), (B), and (D). (A), (B), and (D) as described above can be used:

(A) a quaternary ammonium ion represented by Formula (1) below;
(B) a halide ion; and
(D) water

[Chem. 3]

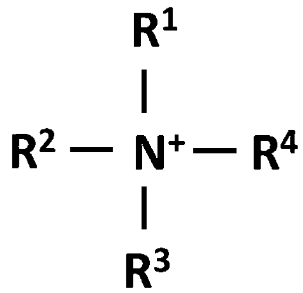

(1)

where $R^1$, $R^2$, $R^3$, and $R^4$ are each independently an alkyl group having carbon number from 1 to 16 (preferably from 1 to 8, and more preferably from 1 to 4), provided that one or more alkyl groups of $R^1$, $R^2$, $R^3$, and $R^4$ have carbon number from 2 to 16 (preferably from 2 to 8, and more preferably from 2 to 4), and $R^1$, $R^2$, $R^3$, and $R^4$ are not all the same alkyl group.

In addition, in a case where the photoresist developer contains a tetramethylammonium ion, the method for manufacturing a photoresist developer can include an electrolysis step A of electrolyzing an aqueous quaternary ammonium halide solution containing a tetramethylammonium ion, the (B), and the (D), and an electrolysis step B of electrolyzing an aqueous quaternary ammonium halide solution containing the (A), the (B), and the (D). An aqueous tetramethylammonium hydroxide solution is obtained by the electrolysis step A, and an aqueous solution of a hydroxide of the quaternary ammonium ion represented by Formula (1) is obtained by the electrolysis step B. Then, the obtained aqueous tetramethylammonium hydroxide solution and the aqueous solution of the hydroxide of the quaternary ammonium ion represented by Formula (1) are mixed, whereby a photoresist developer can be obtained.

In the manufacturing of the photoresist developer by electrolysis, it is possible to use an electrolytic cell in which one or more cation exchange membranes are disposed between an anode and a cathode and which is provided with a raw material chamber for supplying an aqueous quaternary ammonium halide solution as a raw material and a base chamber (cathode chamber) in which an aqueous quaternary ammonium hydroxide solution is produced. In the manufacturing

method using this electrolytic cell, quaternary ammonium ions contained in the aqueous quaternary ammonium halide solution pass through the cation exchange membrane disposed on the cathode side during electrolysis. As a result, an aqueous quaternary ammonium hydroxide solution is produced in the base chamber.

The above example is an example in which one or more cation exchange membranes are disposed. However, an electrolytic cell in which a plurality of membranes selected from the group consisting of a cation exchange membrane, an anion exchange membrane, and a bipolar membrane (a composite membrane composed of a cation exchange membrane and an anion exchange membrane) are disposed can be used as long as an aqueous quaternary ammonium hydroxide solution can be produced.

At this time, a halide ion which is an anion of the quaternary ammonium halide moves to the anode side, but a part of the halide ion also moves to the cathode side by diffusion. Therefore, depending on the concentration of the liquid used as the raw material, about from 1 to several tens ppm of halide ions may be mixed into the aqueous quaternary ammonium hydroxide solution.

The method for manufacturing a photoresist developer can include, before the electrolysis step, a preparation step of preparing an aqueous quaternary ammonium halide solution to be used as a raw material in the electrolysis step.

The preparation step is not particularly limited. For example, an aqueous quaternary ammonium halide solution can be prepared by manufacturing an aqueous quaternary ammonium halide solution. A method for manufacturing the aqueous quaternary ammonium halide solution is not particularly limited. For example, from the viewpoint of productivity and quality, it is preferably a step of producing a quaternary ammonium halide by reacting a trialkylamine with an alkyl halide in ultrapure water.

The trialkylamine and alkyl halide used as raw materials are not particularly limited as long as the quaternary ammonium ion represented by Formula (1) can be produced. Specifically, among the alkyl groups contained in the trialkylamine and the alkyl groups contained in the alkyl halide, one or more alkyl groups are alkyl groups having carbon number from 2 to 16 (preferably from 2 to 8, and more preferably from 2 to 4), and the other alkyl groups are alkyl groups having carbon number from 1 to 16 (preferably from 1 to 8, and more preferably from 1 to 4).

In addition, in a case where the photoresist developer contains a tetramethylammonium ion, the trialkylamine can contain trimethylamine, and the alkyl group contained in the alkyl halide can be a methyl group.

The halide ion which is an anion of an alkyl halide can be the same as (B) described above.

Since impurities contained in the quaternary ammonium halide are also diffused and mixed into the aqueous quaternary ammonium hydroxide solution, it is preferable to use a highly pure quaternary ammonium halide. Specifically, the concentration of the alcohol in the aqueous quaternary ammonium halide solution is preferably 0.1 to 10000 mass ppm, and more preferably 0.1 to 100 mass ppm. The concentration of the amine is preferably 0.1 to 10000 mass ppm, and more preferably 0.1 to 100 mass ppm.

The photoresist developer can develop the exposed photoresist layer. That is, the photoresist development method according to the present invention can include a development step of developing the exposed photoresist layer using the photoresist developer according to the present invention. As the development step, a step similar to the development step in a method for manufacturing a semiconductor device to be described below can be used.

As described below, the photoresist layer contains a photosensitive resin. That is, the photoresist developer is preferably a developer for developing an exposed photoresist layer containing a photosensitive resin. As the photosensitive resin, a polymer contained in the photosensitive resin can have photosensitivity, or the photosensitive resin can have photosensitivity by containing a photosensitizer. For example, a polymer contained in the photosensitive resin can be chemically or structurally changed by exposure, a photosensitizer that can be contained in the photosensitive resin can be chemically or structurally changed by exposure, or the photosensitive resin can be chemically or structurally changed by a change in a photosensitive material (chemically amplified photoresist). A known photosensitizer can be used.

Also, an aspect of the present invention is the use of a photoresist developer for developing a photoresist layer containing a photosensitive resin.

Hereinafter, a method for manufacturing a semiconductor device, which is an aspect of the present invention, will be described.

The manufacture of a semiconductor device using the photoresist developer according to the present invention can be carried out in accordance with a known method.

For example, a method for manufacturing a semiconductor device includes an exposure step of irradiating a photoresist layer on a substrate with light through a photomask having a predetermined pattern; and

a development step of developing the photoresist layer exposed in the exposure step and forming a resist pattern corresponding to the pattern of the photomask, wherein
in the development step, the exposed photoresist layer is developed using the photoresist developer.

In addition to the above steps, the method for manufacturing a semiconductor device can include the following steps.

For example, a forming step of forming a photoresist layer by applying a coating liquid containing a photosensitive resin to a predetermined substrate surface and drying the coating liquid is exemplified. The drying temperature in the forming step is not particularly limited, and is, for example, from 50 to 190°C, and preferably from 70 to 160°C. The drying time is not particularly limited, and is, for example, from 30 to 150 seconds, and preferably 60 to 120 seconds.

The coating liquid contains, for example, a photosensitive resin and a solvent. The solvent is not particularly limited as long as it can dissolve the photosensitive resin, and for example, an ester-based solvent such as propylene glycol monomethyl ether acetate, or a ketone-based solvent can be used.

The method for applying the coating liquid is not particularly limited, a known method can be used, and examples thereof include a spin coating method.

The thickness of the photoresist layer is not particularly limited, and is preferably, for example, from 1 nm to 20 μm.

The method for manufacturing a semiconductor device can include, after the development step, a washing step of washing and removing the developer containing an eluted resist component using a rinse liquid, and a drying step of drying the semiconductor device obtained in the washing step. The washing time in the washing step is not particularly limited, and is, for example, from 10 to 120 seconds, and preferably from 10 to 90 seconds. The temperature in the washing step is not particularly limited, and is, for example, from 10 to 40°C. As the rinse liquid, for example, known water such as ion-exchanged water can be used.

The drying temperature in the drying step is not particularly limited, and is, for example, from 50 to 100°C. The drying time is not particularly limited, and is, for example, from 30 to 150 seconds.

In addition, in a case where a chemically amplified photoresist is used, it is preferable that the method for manufacturing a semiconductor device includes a heating step of heating the photoresist layer as necessary to promote generation of an acid. The heating temperature is not particularly limited, and is, for example, from 70 to 110°C. This heating step is performed after the exposure step. The heating time in the heating step is not particularly limited, and is from 1 to 10 minutes.

In the exposure step, the photoresist layer on the substrate is irradiated with light through a photomask having a predetermined pattern. The wavelength of the light is preferably a wavelength corresponding to the luminous sensitivity of the photoresist. In a case of forming a fine resist pattern, light having a short wavelength such as excimer laser light is irradiated. Exposure by electron beam or X-ray irradiation is also possible depending on the type of the photoresist. Examples of the light source include a mercury lamp used for g-line (436 nm), h-line (405 nm), and i-line (365 nm), an excimer laser used for KrF (248 nm) and ArF (193 nm), an EUV (13.5 nm) light source, and an electron-beam light source. That is, the photoresist developer can be a photoresist developer for developing a photoresist layer exposed using one or more light sources selected from the group consisting of a mercury lamp, an excimer laser, an EUV light source, and an electron-beam light source, or can be a photoresist developer for developing a photoresist layer exposed using one or more excimer lasers selected from the group consisting of a KrF excimer laser and an ArF excimer laser.

An aspect of the present invention is the use of a photoresist developer for developing an exposed photoresist layer. The light source described above can be used for the exposure. For example, the aspect can be the use of a photoresist developer to develop a photoresist layer exposed using an excimer laser. Further, the aspect is preferably the use of a photoresist developer for developing a photoresist layer exposed using one or more excimer lasers selected from the group consisting of a KrF excimer laser and an ArF excimer laser.

The photoresist layer is a layer containing a photosensitive resin (photoresist). The photoresist is not particularly limited, and can be a positive photoresist or a negative photoresist. The positive photoresist is a photosensitive resin in which an exposed region is solubilized in a developer, and the negative photoresist is a photosensitive resin in which an exposed region is insolubilized in a developer.

Examples of the positive photoresist include (meth)acrylic resins such as polymethyl (meth)acrylate, urethane resins such as polyurethane, phenol resins such as a novolac resin, styrene resins such as a polyhydroxystyrene (PHS) resin, and resins having an aliphatic polycyclic skeleton. Among them, one or more resins selected from the group consisting of a (meth)acrylic resin and a styrene resin are preferable. One of these resins can be used alone, or two or more thereof may be used in combination.

Examples of the negative photoresist include polyvinyl cinnamate and a mixture of a resin having an unsaturated carbon bond and an azide compound.

As described above, examples of the styrene resin include a polyhydroxystyrene resin.

In one aspect of the photoresist containing a polyhydroxystyrene resin, it is preferable that a part of the hydroxyl groups of the polyhydroxystyrene resin contained in the photoresist is substituted with a protective group. The introduction of the protective group makes the photoresist insoluble in the photoresist developer in the unexposed region. In the exposed portion, a chemical reaction occurs in which the protective group is eliminated and replaced with a hydroxyl group, which makes the photoresist soluble in the photoresist developer. At the time, when the amount of substance of the hydroxyl groups of the polyhydroxystyrene resin contained in the photoresist is defined as 100 mol, the amount of substance of the protective groups in the polyhydroxystyrene resin is preferably from 75 to 300 mol, and more preferably

from 90 to 200 mol.

The protective group is not particularly limited, and examples thereof include an acetal group, a ketal group, a silyl group, a silyl ether group, and a tertiary alkyl ester group. The protective group can have an arbitrary substituent or can have a cyclic structure. Specific examples thereof include a tert-butoxycarbonyl group, an isopropoxycarbonyl group, and a benzyloxycarbonyl group. Among these, a tert-butoxycarbonyl group is preferable.

In a dissolution rate evaluation test of the photoresist layer containing the polyhydroxystyrene resin at a temperature of 23°C, the ratio of the hydroxyl group and the tert-butoxycarbonyl group in the polyhydroxystyrene resin is 70 : 30 on a molar basis, the thickness of the photoresist layer is 200 nm, and the dissolution rate of the photoresist layer in the case of using the photoresist developer according to an embodiment of the present invention is preferably 110% or more, and more preferably 130% or more, with respect to the dissolution rate of the photoresist layer in the case of using a photoresist developer containing tetramethylammonium hydroxide. The fact that the dissolution rate of the photoresist layer in the case of using the photoresist developer according to an embodiment of the present invention is within the above range indicates that the solubility of the photoresist in the vicinity of the boundary of the photomask where the chemical reaction by light is less likely to proceed is excellent. As a result, the pattern shape is likely to be good. Although the specific method of the dissolution rate evaluation test will be described below, the temperature of the dissolution rate evaluation test indicates the temperature of the photoresist developer to be evaluated.

The upper limit of the dissolution rate is not particularly limited, and is, for example, from 110 to 500%, from 110 to 300%, or from 130% to 200%.

The dissolution rate can be adjusted by changing the carbon numbers of alkyl groups $R^1$ to $R^4$ in the quaternary ammonium ion represented by Formula (1). For example, when a compound in which any three of $R^1$, $R^2$, $R^3$, and $R^4$ are methyl groups and the remaining one alkyl group has carbon number from 2 to 16 is used, the dissolution rate is likely to be increased. When the carbon number of the alkyl group as the lipophilic moiety is too large, the dissolution rate of the resist layer is lowered due to adsorption inhibition, and thus, the carbon number of the alkyl group as the lipophilic moiety is 16 or less. The carbon number of the alkyl group is preferably 8 or less, and more preferably 4 or less. When any three of $R^1$, $R^2$, $R^3$ and $R^4$ are methyl groups, a hydrophilic moiety is formed in contrast to the lipophilic moiety, and thus, the wettability with the photoresist resin is improved, whereby the dissolution rate can be increased, which is preferred.

As described above, examples of the photoresist include a (meth)acrylic resin. The structure of the (meth)acrylic resin is not particularly limited, but the (meth)acrylic resin preferably has a monomer unit represented by the following Formula (D). Monomer units represented by the following Formula (D) can be the same monomer units or different monomer units.

[Chem. 4]

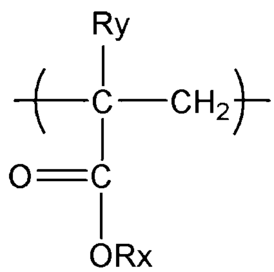

(D)

In Formula (D), Rx represents an arbitrary protective group, and Ry represents hydrogen or a methyl group.

The protective group represented by Rx is not particularly limited as long as it is a functional group that is eliminated by exposure, and examples thereof include a hydrocarbon group having carbon number from 3 to 12 (preferably carbon number from 6 to 9). The hydrocarbon group may be linear, branched, or cyclic. Among them, an alicyclic hydrocarbon group is preferable.

The content of the monomer unit represented by Formula (D) in the (meth)acrylic resin is not particularly limited, but can be from 50 to 100 parts by mass, or can be from 70 to 100 parts by mass.

Specifically, a tert-butoxycarbonyl group, a 2-alkyl-substituted adamantyloxycarbonyl group such as a 2-methyladamantyloxycarbonyl group, a 2-alkyl-substituted norbornyloxycarbonyl group such as a 2,6-carbolactonenorbornyl group, and a 2-pyranyloxycarbonyl group are preferable.

In the development step, the photoresist layer exposed in the exposure step is developed and a resist pattern corresponding to the pattern of the photomask is formed. In the method for manufacturing a semiconductor device according to an embodiment of the present invention, the exposed photoresist layer is developed using the photoresist developer. That is, a resist pattern is formed on the substrate, and a region covered with the resist pattern and a region not covered with the resist pattern are formed.

The development of the photoresist layer is preferably carried out by, for example, a method of applying the

photoresist developer according to an embodiment of the present invention to the photoresist layer exposed in the exposure step.

The development of the exposed photoresist layer is not particularly limited except that the photoresist developer is used, and for example, a known method such as an immersion method, a puddle development method, or a spray development method can be adopted without any particular limitation. The immersion method is a development method in which a substrate such as a silicon wafer on which a photoresist layer is formed is immersed in a developer for a certain period of time, and then immersed in pure water and dried. The puddle development method is a development method in which a developer is dropped on a photoresist layer, and the photoresist layer is left to stand for a certain period of time, and then washed with pure water and dried. The spray development method is a development method in which a developer is sprayed onto a photoresist layer, and then the photoresist layer is washed with pure water and dried.

At this time, when the photoresist is a positive photoresist, the exposed region of the photoresist layer exposed in the exposure step is removed. On the other hand, when the photoresist is a negative photoresist, the unexposed region of the photoresist layer that is not exposed in the exposure step is removed.

The temperature of the development step is not particularly limited, and is, for example, 10 to 40°C, and preferably 15 to 30°C. The time of the development step is not particularly limited, and is, for example, 10 to 120 seconds, and preferably 10 to 90 seconds.

Note that the term "semiconductor substrate" is used in the process of manufacturing a semiconductor device.

The method for manufacturing a semiconductor device preferably includes a processing step of processing a region of the substrate having the resist pattern on the surface, the region not being covered with the resist pattern. The processing step is a step after the development step. When the method for manufacturing a semiconductor device includes the processing step, a semiconductor device having a substrate on which a pattern is formed can be obtained. As a processing method, a known method can be used, and examples thereof include etching processing such as wet etching and dry etching, and plating processing.

Examples

Hereinafter, to specifically describe the present invention, examples will be shown, but the present invention is not limited to these examples. The evaluations performed in Examples and Comparative Examples were determined by the following methods.

(Examples 1 to 7 and Comparative Examples 1 to 3)

[Photoresist Developer]

Each aqueous quaternary ammonium hydroxide solution was prepared to 0.26 mol/L to obtain a photoresist developer. The dissolution rate of the photoresist was evaluated by the method described below.

- Ethyltrimethylammonium hydroxide (ETMAH, made from raw materials)
- Propyltrimethylammonium hydroxide (PTMAH, made from raw materials)
- Butyltrimethylammonium hydroxide (BTMAH, made from raw materials)
- Tetramethylammonium hydroxide (TMAH, available from Tokuyama Corporation)
- Tetraethylammonium hydroxide (TEAH, available from Tokyo Chemical Industry Co., Ltd.)

[Surfactant]

- SURFYNOL (registered trademark) 465 (available from Nisshin Chemical Industry Co., Ltd.)

[Preparation of Aqueous Quaternary Ammonium Hydroxide Solution]

ETMAH, PTMAH, and BTMAH were prepared by the following procedure. An aqueous quaternary ammonium halide solution was produced from trialkylamine and alkyl halide as raw materials, and an aqueous quaternary ammonium hydroxide solution was produced by electrolysis using the aqueous quaternary ammonium halide solution as a raw material. Specifically, in the case of ETMAH, trimethylamine and ethyl chloride at a molar ratio of 1:1 were put into a reaction vessel containing ultrapure water, which were retained at a reaction temperature of 60°C to produce a 50 mass% aqueous ethyltrimethylammonium chloride solution. Trimethylamine and ethyl chloride, which were unreacted components, and ethanol, which was a by-product, were each removed under reduced pressure until the amounts thereof were

each less than 100 ppm, and then ETMAH was produced using the electrolytic cell shown in FIG. 1. In FIG. 1, 1 denotes an anode, 2 denotes a cathode, 3 denotes a power supply, 4 denotes an anode chamber, 5 denotes a raw material chamber, 6 denotes an intermediate chamber, 7 denotes a cathode chamber, 8 denotes an anion exchange membrane, and 9 denotes a cation exchange membrane.

A platinum-plated nickel plate was used as the cathode, a platinum-plated titanium plate was used as the anode, two-sheets of Nafion N324 (available from The Chemours Company) were used as the cation exchange membrane, and ASE (available from ASTOM CORPORATION) was used as the anion exchange membrane. Electrolysis was continuously carried out while gradually increasing the current density and finally maintaining the current density at 30 A/$dm^2$ and the temperature at 40°C, by circulating 0.5 N hydrochloric acid in the anode chamber, a 50 mass% aqueous ethyltrimethylammonium chloride solution in the raw material chamber between the anion exchange membrane and the cathode-side cation exchange membrane, and ultrapure water in the intermediate chamber between the cathode chamber and the two cation exchange membranes. During the electrolysis, the aqueous ethyltrimethylammonium chloride solution was replenished, and the concentration of ethyltrimethylammonium chloride in the raw material chamber was maintained at 40 mass% or more. When the concentration of ETMAH in the cathode chamber increased to 30 mass%, the electrolysis was terminated, resulting in the formation of an aqueous ETMAH solution. This aqueous ETMAH solution was diluted with ultrapure water and used in Example 1 at the concentration shown in Table 1.

PTMAH and BTMAH were prepared in the same manner as in the preparation of ETMAH except that propyl chloride and butyl chloride were used as alkyl halides, respectively, and made into the concentrations shown in Table 1, and the prepared PTMAH and BTMAH were used in Examples 2 to 7. The surfactant shown in Table 1 was added in Examples 6 to 7 and Comparative Example 3.

The concentrations of the amines in the photoresist developers of Examples 1 to 7 and Comparative Examples 1 to 3 were all less than 100 mass ppm. Further, the concentrations of the alcohols in the photoresist developers of Examples 1 to 7 and Comparative Examples 1 to 3 were all less than 100 mass ppm. In addition, calcium ions, sodium ions, potassium ions, chromium ions, nickel ions, iron ions, lead ions, and aluminum ions in the photoresist developers of Examples 1 to 7 and Comparative Examples 1 and 3 were all 0.1 ppb or less. The photoresist developer of Comparative Example 2 was purified with a cation exchange resin to reduce all of calcium ions, sodium ions, potassium ions, chromium ions, nickel ions, iron ions, lead ions, and aluminum ions in the photoresist developer to 0.1 ppb or less.

[Evaluation Method]

1) Measurement of Halide Ion Concentration

In a case where the quaternary ammonium hydroxide concentration of the photoresist developer was 5 mass% or less, the developer was allowed to pass through an Ongard Cartridge IIH+ type 2.5 cc (available from Thermo Fisher Scientific Inc.) as it was, and in a case where the concentration exceeded 5 mass%, the developer was diluted with ultrapure water to 5 mass% and allowed to pass through the Ongard Cartridge IIH+ type 2.5 cc. Thereafter, the liquid was recovered, and anion analysis of the recovered liquid was performed by an ion chromatograph (Integrion, available from Thermo Fisher Scientific Inc.) to determine a halide ion concentration.

2) Hydroxide Ion Concentration

One mL of the developer prepared in each of Examples and Comparative Examples was subjected to neutralization titration with 0.1 mol/L hydrochloric acid using a Hiranuma automatic titrator (COM-1700, available from HIRANUMA). The hydroxide ion concentration of the photoresist developer was determined from the liquid amount of 0.1 mol/L hydrochloric acid required for neutralization.

3) Dissolution Rate Evaluation of Photoresist Layer

The dissolution rate of the photoresist layer in the photoresist developer was evaluated using a quartz crystal microbalance (QCM) method.

A photoresist resin described below was spin-coated on a QCM substrate and pre-baked at 90°C for 90 seconds to produce a photoresist layer having a thickness of 200 nm. The photoresist layer was immersed in a photoresist developer at a temperature of 23°C, and the dissolution rate of the photoresist layer was evaluated. The dissolution rate was determined by the time required for an impedance value obtained by the QCM method to turn from increase to decrease. The viscosity of the interface decreases when the photoresist layer is completely dissolved, and thus, it can be determined that the shorter the time for the impedance to turn to decrease, the higher the dissolution rate of the photoresist layer.

As the photoresist resin, a liquid adjusted in advance with a resin composition before exposure and a liquid

adjusted in advance with a resin composition after exposure were used. Specifically, a resin (30% t-Boc-PHS) in which the hydroxyl group (OH group) of polyhydroxystyrene was protected with a tert-butoxycarbonyl group (t-Boc group) and substituted in such a manner that OH group : t-Boc group = 70:30 was satisfied on a molar basis was prepared, and a liquid adjusted by dissolving the resin in propylene glycol monomethyl ether acetate (PGMEA) was used.

The dissolution rate was evaluated in accordance with the following evaluation criteria with reference to 2.4 mass% (0.26 M) TMAH. The results are shown in Table 1 as "Dissolution rate".

(Evaluation Criteria)

A: 130% or more of dissolution rate of TMAH
B: 110% or more and less than 130% of dissolution rate of TMAH
C: 90% or more and less than 110% of dissolution rate of TMAH
D: Less than 90% of dissolution rate of TMAH

4) Evaluation of Development Characteristics

The development characteristics were evaluated by forming patterns on different resists A and B and evaluating the shapes thereof.

[Pattern Formation Using Resist A]

- Polyhydroxystyrene (weight average molecular weight: 10000) in which 50 mol% of hydrogen atoms of hydroxyl groups are substituted with tert-butoxycarbonyl groups ··· 100 parts by mass
- Triphenylsulfonium hexafluoroantimonate ··· 5 parts by mass
- Triethanolamine ··· 0.2 parts by mass
- Propylene glycol monomethyl ether acetate (PGMEA) ··· 400 parts by mass

The prepared resist A was spin-coated on a silicon wafer treated with hexamethyldisilazane using a spin coater, and baked at 90°C for 90 seconds using a hot plate to obtain a resist film having a film thickness of 0.6 μm. The film was exposed to a KrF excimer laser through a mask pattern (1:1 line-and-space of line width 260 nm) using a KrF exposure apparatus. Thereafter, post-exposure baking was performed at 110°C for 60 seconds. Further, the resist film was developed with an aqueous solution of quaternary ammonium hydroxide having a concentration shown in Table 1 at 23°C for 30 seconds, rinsed with ultrapure water for 30 seconds, and dried to obtain a pattern shape.

[Pattern Formation Using Resist B]

[Chem. 5]

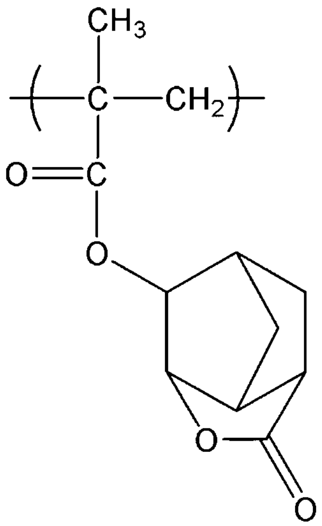

(2-1)

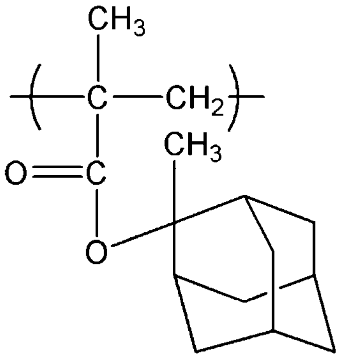

(2-2)

- Copolymer (weight average molecular weight: 14000) containing a monomer unit represented by the above Formula (2-1) and a monomer unit represented by the above Formula (2-2) at a molar ratio of 50 : 50 ··· 100 parts by mass,
- Triphenylsulfonium trifluoromethanesulfonate ··· 1.5 parts by mass
- Triethanolamine ··· 0.1 parts by mass

The above materials were dissolved in a mixed solvent of 950 parts by mass of propylene glycol monomethyl ether acetate (PGMEA) and 100 parts by mass of γ-butyrolactone to obtain a positive resist solution. Then, the resist solution was applied onto a silicon wafer using a spin coater, and dried on a hotplate at 140°C (pre-bake) for 90 seconds to form a resist layer of 250 nm. Subsequently, the resist layer was irradiated with ArF excimer laser light (193 nm) using an ArF exposure apparatus through a mask pattern (1:1 line-and-space of line width of 100 nm). Thereafter, the resist layer was subjected to a heating treatment (post-exposure bake: PEB) at 130°C for 90 seconds. Subsequently, the resist film was subjected to puddle development for 20 seconds with an aqueous solution of quaternary ammonium hydroxide having a concentration shown in Table 1, washed with water for 60 seconds, and dried.

[Evaluation of Shape]

The line-and-space state obtained by the formation of the pattern was observed with a scanning electron microscope (JSM-7800F Prime, available from JEOL Ltd.), the width of the exposed bottom portion was observed at 10 points, and the arithmetic average value of the observed widths was determined. Then, the dimensional difference of the arithmetic average value of the observed widths with respect to the size of the mask pattern was calculated by the following equation. The evaluation was made in accordance with the following criteria. The results are shown in Table 1 as "development shape".

(Dimensional difference (%)) = (size (nm) of mask pattern - arithmetic average value (nm) of width)/size (nm) of mask pattern $\times$ 100

(Evaluation Criteria)

A: Dimensional difference of less than 3%
B: Dimensional difference of 3% or more and less than 5%
C: Dimensional difference of 5% or more and less than 10%
D: Dimensional difference of 10% or more, or resist remains and bottom cannot be seen

5) Surface Tension

The surface tension of the developer prepared in each of Examples and Comparative Examples at 25°C was measured using a surface tensiometer DY-300 (available from Kyowa Interface Science Co., Ltd.). The measurement was performed in accordance with "Test Method by Wilhelmy Surface Tensiometer" of JIS2241.

(Example 8)

The development characteristics of the resist A were evaluated using the same photoresist developer as in Example 1 except that the Cl concentration of Example 1 was changed to 210 ppm. As a result, the developed shape was evaluated as B as in Example 1, but the proportion of the resist residue portion was increased as compared with Example 1.

(Example 9)

The development characteristics of the resist A were evaluated using the same photoresist developer as in Example 2 except that the Cl concentration in Example 2 was changed to 85 ppm. As a result, the developed shape was evaluated as A as in Example 1, but the proportion of the resist residue portion was increased as compared with Example 2.

[Table 1]

| | Quaternary ammonium hydroxide | | TMAH | Surfactant | | Hydroxide ion | Water | Halide ion | | Dissolution rate | Development characteristics | | Surface tension |
|---|---|---|---|---|---|---|---|---|---|---|---|---|---|
| | Type | mass % | mass % | Type | mass ppm | mass% | mass% | Type | mass ppm | | Resist A | Resist B | mN/m |
| Example 1 | ETMAH | 2.7 | - | - | - | 0.44 | 97.3 | Cl | 0.03 | B | B | B | 72.8 |
| Example 2 | PTMAH | 3.1 | - | - | - | 0.44 | 96.9 | Cl | 0.5 | A | A | A | 71.9 |
| Example 3 | BTMAH | 3.5 | - | - | - | 0.44 | 96.5 | Cl | 0.3 | A | A | A | 70.6 |
| Example 4 | BTMAH | 1.7 | 1.2 | - | - | 0.44 | 97.1 | Cl | 0.2 | - | A | A | - |
| Example 5 | BTMAH | 0.4 | 2.1 | - | - | 0.44 | 97.5 | Cl | 0.1 | - | B | B | - |
| Example 6 | BTMAH | 0.4 | 2.1 | SURFYNOL 465 | 20 | 0.44 | 97.5 | Cl | 0.1 | - | A | A | - |
| Example 7 | PTMAH | 0.4 | 2.1 | SURFYNOL 465 | 20 | 0.44 | 97.5 | Cl | 0.2 | - | A | A | - |
| Comparative Example 1 | - | - | 2.4 | - | - | 0.44 | 97.6 | Cl | 0.1 | C | C | C | 73.2 |
| Comparative Example 2 | TEAH | 3.8 | - | - | - | 0.44 | 96.2 | Cl | 4 | D | D | D | - |
| Comparative Example 3 | - | - | 2.4 | SURFYNOL 465 | 20 | 0.44 | 97.6 | Cl | 0.1 | - | C | C | - |

In the table, "-" in the dissolution rate column and the surface tension column indicates that the measurement was not performed.

As shown in Table 1, the results were that ETMAH, PTMAH, and BTMAH had a higher dissolution rate than that of TMAH, and had excellent development characteristics. During development, the protective group was likely to remain in the vicinity of the mask of the exposed portion, and in a case of only TMAH, the side surface of the exposed portion remained undissolved, and the width of the exposure bottom portion became narrower than the line width of the mask pattern. On the other hand, in ETMAH, PTMAH, and BTMAH, the amount of the undissolved residue on the side surface of the exposed portion was small, the width of the exposure bottom portion was close to the line width of the mask pattern, and it was possible to obtain a pattern having a more rectangular shape and having a side surface nearly perpendicular to the bottom portion. In addition, in a case of mixing BTMAH and TMAH as well, the amount of the undissolved residue on the side surface portion was smaller than that in the case of using only TMAH, and a more rectangular pattern was obtained. It was also found that the undissolved residue was further reduced in a case of adding a small amount of a surfactant.

Industrial Applicability

The photoresist developer according to the present invention can be used as a photoresist developer providing a good pattern shape.

Reference Signs List

1: Anode
2: Cathode
3: Power source
4: Anode chamber
5: Raw material chamber
6: Intermediate chamber
7: Cathode chamber
8: Anion-exchange membrane
9: Cation-exchange membrane

**Claims**

1. A photoresist developer comprising the following (A), (B), (C) and (D):

(A) a quaternary ammonium ion represented by Formula (1) below;
(B) a halide ion;
(C) a hydroxide ion; and
(D) water

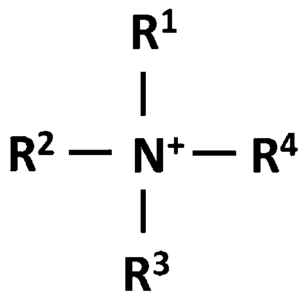

(1)

where $R^1$, $R^2$, $R^3$, and $R^4$ are each independently an alkyl group having carbon number from 1 to 16, provided that one or more alkyl groups of $R^1$, $R^2$, $R^3$, and $R^4$ have carbon number from 2 to 16, and $R^1$, $R^2$, $R^3$, and $R^4$ are not all the same alkyl group.

2. The photoresist developer according to claim 1, wherein the photoresist developer has a surface tension of 73.0 mN/m

or less at 25°C.

3. The photoresist developer according to claim 1 or 2, wherein $R^1$, $R^2$, and $R^3$ in Formula (1) are methyl groups, and $R^4$ in Formula (1) is an alkyl group having carbon number from 2 to 16.

4. The photoresist developer according to any one of claims 1 to 3, wherein the (B) halide ion is a chloride ion or a bromide ion.

5. The photoresist developer according to any one of claims 1 to 4, wherein the halide ion is in an amount of from 0.0000001 to 0.01 parts by weight when the hydroxide ion is in an amount of 1 part by weight, in the photoresist developer.

6. The photoresist developer according to any one of claims 1 to 5, further comprising one or more compounds selected from the group consisting of an alcohol and an amine.

7. The photoresist developer according to any one of claims 1 to 6, the photoresist developer being a photoresist developer for developing an exposed photoresist layer, the photoresist layer comprising a photosensitive resin.

8. The photoresist developer according to any one of claims 1 to 7, wherein the photoresist developer comprises one or more metal ions selected from the group consisting of a calcium ion, a sodium ion, a potassium ion, a chromium ion, a nickel ion, an iron ion, a lead ion, and an aluminum ion, and a content of each of the metal ions in the photoresist developer is independently 1 ppb or less.

9. The photoresist developer according to any one of claims 1 to 8, the photoresist developer being a photoresist developer for developing a photoresist layer exposed using one or more excimer lasers selected from the group consisting of a KrF excimer laser and an ArF excimer laser.

10. The photoresist developer according to any one of claims 1 to 9, wherein

in a dissolution rate evaluation test at 23°C of a photoresist layer containing a polyhydroxystyrene resin,
a ratio of a hydroxyl group and a tert-butoxycarbonyl group in the polyhydroxystyrene resin is 70 : 30 on a molar basis,
the photoresist layer has a thickness of 200 nm, and
a dissolution rate of the photoresist layer in a case of using the photoresist developer is 110% or more with respect to the dissolution rate of the photoresist layer in a case of using a photoresist developer containing tetramethylammonium hydroxide.

11. The photoresist developer according to any one of claims 1 to 10, further comprising a surfactant, wherein a concentration of the surfactant in the photoresist developer is less than 100 mass ppm.

12. The photoresist developer according to any one of claims 1 to 11, further comprising a tetramethylammonium ion.

13. A photoresist development method, the method comprising a development step of developing an exposed photoresist layer using the photoresist developer according to any one of claims 1 to 12.

14. A method for manufacturing a semiconductor device, the method comprising:

an exposure step of irradiating a photoresist layer on a substrate with light through a photomask having a predetermined pattern; and
a development step of developing the photoresist layer exposed in the exposure step and forming a resist pattern corresponding to the pattern of the photomask, wherein
in the development step, the exposed photoresist layer is developed using the photoresist developer according to any one of claims 1 to 12.

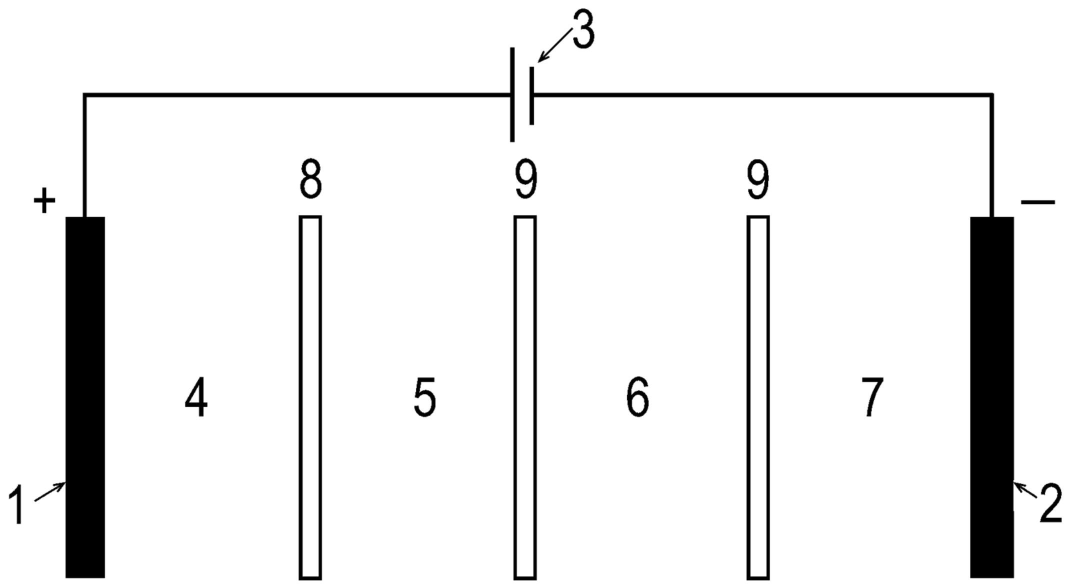


FIG.1

## INTERNATIONAL SEARCH REPORT

International application No.
**PCT/JP2024/031290**

### A. CLASSIFICATION OF SUBJECT MATTER

***G03F 7/32***(2006.01)i; ***C07C 211/63***(2006.01)i; ***G03F 7/20***(2006.01)i; ***G03F 7/26***(2006.01)i; ***H01L 21/304***(2006.01)i; ***H01L 21/306***(2006.01)i; ***H01L 21/308***(2006.01)i
FI: G03F7/32; C07C211/63; G03F7/20 501; G03F7/20 521; G03F7/26 501; H01L21/304 647A; H01L21/306 B; H01L21/308 B

According to International Patent Classification (IPC) or to both national classification and IPC

### B. FIELDS SEARCHED

Minimum documentation searched (classification system followed by classification symbols)

G03F7/32; C07C211/63; G03F7/20; G03F7/26; H01L21/304; H01L21/306; H01L21/308

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| X | JP 2020-122983 A (FUJIFILM CORPORATION) 13 August 2020 (2020-08-13) claims | 1-14 |

☐ Further documents are listed in the continuation of Box C.  ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **30 September 2024** | **19 November 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (July 2022)

**INTERNATIONAL SEARCH REPORT**
**Information on patent family members**

International application No.
**PCT/JP2024/031290**

| Patent document cited in search report | Publication date (day/month/year) | Patent family member(s) | Publication date (day/month/year) |
|---|---|---|---|
| JP 2020-122983 A | 13 August 2020 | US 2019/0025702 A1 claims<br>TW 201807511 A<br>CN 108885412 A<br>KR 10-2018-0126555 A | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP H04226466 A